# EUROPEAN PATENT SPECIFICATION

(11) **EP 4 289 968 B1**
(45) Date of publication and mention of the grant of the patent: **12.08.2026**
(21) Application number: 21952230.7
(22) Date of filing: 04.08.2021
(51) Int. Cl.: C12Q 1/6883, C12Q 1/6851, C12N 15/11

(54) **PRIMER-PROBE SET AND KIT FOR HUMAN LEUKOTRIENE RECEPTOR CYSLTR1 MRNA RT-PCR DETECTION**
PRIMER-SONDENSET UND KIT FÜR DEN NACHWEIS VON MRNA-RT-PCR DES MENSCHLICHEN LEUKOTRIENREZEPTORS CYSLTR1
ENSEMBLE AMORCE-SONDE ET KIT POUR LA DÉTECTION PAR RT-PCR D'ARNM DU RÉCEPTEUR DES LEUCOTRIÈNES HUMAINS CYSLTR1

(43) Date of publication of application: 13.12.2023
(73) Proprietor: Hangzhou Zheda Dixun Biological Gene Engineering Co., Ltd, Hangzhou, Zhejiang 310052 (CN)
(72) Inventor: LIU, Yi, Zhejiang 310052 (CN); WU, Zhoujie, Zhejiang 310052 (CN); WU, Shandong, Zhejiang 310052 (CN); CHENG, Lei, Zhejiang 310052 (CN); JIANG, Xuehan, Zhejiang 310052 (CN); LEI, Wei, Zhejiang 310052 (CN); QIAN, Lei, Zhejiang 310052 (CN); YANG, Xukai, Zhejiang 310052 (CN); WANG, Meijie, Zhejiang 310052 (CN); WANG, Jiping, Zhejiang 310052 (CN)
(74) Representative: Bayramoglu et al.
(86) International application number: PCT/CN2021/110524
(87) International publication number: WO 2023/010328

(56) References cited:
- WO-A2-2006/024449
- US-A1- 2006 269 949
- US-A1- 2017 165 309
- US-B1- 6 465 212
- WEI SONG ET AL: "Antagonism of cysteinyl leukotriene receptor 1 (cysLTR1) by montelukast suppresses cell senescence of chondrocytes", CYTOKINE, vol. 103, 1 March 2018 (2018-03-01), US, pages 83 - 89, XP055584474, ISSN: 1043-4666, DOI: 10.1016/j.cyto.2017.12.021
- MITA H ET AL: "Levels of cysteinyl leukotriene receptor mRNA in human peripheral leucocytes: significantly higher expression of cysteinyl leukotriene receptor 2 mRNA in eosinophils", CLINICAL & EXPERIMENTAL ALLERGY, WILEY INTERSCIENCE, UK, vol. 31, no. 11, 7 July 2008 (2008-07-07), pages 1714 - 1723, XP071884245, ISSN: 0954-7894, DOI: 10.1046/J.1365-2222.2001.01184.X
- SHIRASAKI H ET AL: "Agonist- and T"H2 cytokine-induced up-regulation of cysteinyl leukotriene receptor messenger RNA in human monocytes", ANNALS OF ALLERGY, ASTHMA, ELSEVIER, AMSTERDAM, NL, vol. 99, no. 4, 1 October 2007 (2007-10-01), pages 340 - 347, XP026959892, ISSN: 1081-1206, [retrieved on 20071001]
- NEGRI J ET AL: "Corticosteroids as inhibitors of cysteinyl leukotriene metabolic and signaling pathways", JOURNAL OF ALLERGY AND CLINICAL IMMUNOLOGY, ELSEVIER, AMSTERDAM, NL, vol. 121, no. 5, 1 May 2008 (2008-05-01), pages 1232 - 1237, XP022632258, ISSN: 0091-6749, [retrieved on 20080319], DOI: 10.1016/J.JACI.2008.02.007
- STEINKE JOHN W ET AL: "Characterization of interleukin-4-stimulated nasal polyp fibroblasts", AMERICAN JOURNAL OF RESPIRATORY CELL AND MOLECULAR BIOLOGY, AMERICAN LUNG ASSOCIATION, NEW YORK, NY, US, vol. 30, no. 2, 1 February 2004 (2004-02-01), pages 212 - 219, XP002405458, ISSN: 1044-1549, DOI: 10.1165/RCMB.2003-0071OC
- JULIE NEGRI BA ET AL.: "Corticosteroids as inhibitors of cysteinyl leukotriene metabolic and signaling pathways", JOURNAL OF ALLERGY AND CLINICAL IMMUNOLOGY, vol. 121, no. 5, 31 May 2008 (2008-05-31), XP022632258, DOI: 10.1016/j.jaci.2008.02.007
- ZHAO LU, DU XIAOMING; ZHANG YANLING; SUN GUOPING: "Expression and significance of CysLTＲ-1 mＲNA in colon cancer", SHANDONG YIYAO - SHANDONG MEDICAL JOURNAL, SHANDONG SHENG WEISHENGTING,, CN, vol. 58, no. 25, 30 September 2018 (2018-09-30), CN , pages 13 - 15, XP093033036, ISSN: 1002-266X, DOI: 10.3969/j.issn.1002-266X.2018.25.004

## Description

### TECHNICAL FIELD

The present disclosure belongs to the technical field of biological detection, and specifically relates to a primer probe set and a kit for reverse transcription-polymerase chain reaction (RT-PCR) detection of a human leukotriene receptor *CYSLTR1* mRNA.

### BACKGROUND ART

Leukotriene is an unsaturated eicosanoic acid with a conjugated triene structure isolated from the metabolites of an arachidonic acid in leukocytes. The leukotriene can be prepared by the arachidonic acid through catalyzation of a lipoxygenase. Although there is a low content of leukotriene in human body, the leukotriene show very high physiological activity and serves as a chemical mediator that triggers certain allergic reactions, inflammations and cardiovascular diseases. The leukotriene is important in inflammations of the upper and lower respiratory tract. The leukotriene is over 1,000 times stronger than histamine in inducing nasal allergic reactions. The amount of leukotrienes increases significantly in both an immediate reaction stage and a late reaction stage in allergen-induced nasal allergic reactions. Cysteinyl leukotrienes (CysLTs) are inflammatory mediators and regulators in the pathophysiology of asthma and allergic rhinitis (AR), and are key therapeutic targets. The CysLTs can regulate the production of hematopoietic progenitor cells, the recruitment and survival of eosinophils in inflammatory tissues, the activity of cytokines and chemokines, the amount of exhaled NO, the contraction of smooth muscle and the proliferation of fibroblasts.

The biological function of CysLTs depends on the expression of leukotriene receptors on the cell surface. A CysLTs receptor includes a CysLTR1 and a CysLTR2, where the CysLTR1 is a G protein-coupled receptor that is mainly expressed in the spleen, lung, smooth muscle and the like. After being activated by leukotrienes, the CysLTR1 mediates continuous contraction and proliferation of smooth muscle cells, mucosal edema, eosinophil accumulation, and increased mucus secretion, thereby directly leading to the occurrence and development of airway inflammation in the asthma. The CysLTR1 plays a major role in the pathogenesis of asthma. Approved CysLT1 receptor antagonists (such as montelukast, zafirlukast, and pranlukast) act on the CysLTR1 to block the asthma-causing effect. During treatment, leukotriene receptor antagonists (LTRA) can competitively inhibit the binding of leukotrienes to their receptors in the body, and block the activity of CysLTs, thereby inhibiting inflammatory and allergic reactions. However, the curative effect of the LTRA has obvious individual differences, and has a clear positive correlation with the expression level of a leukotriene receptor gene mRNA. By detecting the expression level of *CYSLTRI* mRNA, whether the LTRA has blocked the activity of the CysLTR1 can be determined. This has a certain therapeutic significance for whether the medicine is effective in treating allergic and inflammatory reactions caused by the CysLTR1 pathway.

At present, the detection of the content of CysLTR1 in the body fluids is still detected using an enzyme-linked immunosorbent assay (ELISA) kit. There is no commercial kit for detecting the *CYSLTRI* mRNA. The ELISA method has the problems of small detection range, low sensitivity and unsatisfactory accuracy during the detection.
Wei Song ET AL: "Antagonism of cysteinyl leukotriene receptor 1 (cysLTR1) by montelukast suppresses cell senescence of chondrocytes": Aging is closely associated with osteoarthritis (OA). Although its underlying mechanisms remain unknown, cellular senescence in chondrocytes has become an important therapeutic target for the treatment of OA. Cysteinyl leukotriene receptors (cysLTRs) mediate the pathobiological function of cysteinyl leukotrienes (cysLTs). However, the roles of cysLTRs in the pathogenesis of OA have not been reported before. In the current study, we found that cysLTR1 but not cysLTR2 is expressed in human primary chondrocytes. In addition, stimulation with tumor necrosis factor α (TNF-α) resulted in a significant increase in the expression of cysLTR1. Interestingly, montelukast, a specific cysLTR1 antagonist, attenuated TNF-α-induced up-regulation of the activity of senescence-associated β-galactosidase (SA-β-Gal). In addition, TNF-α led to cell cycle arrest at the G0/G1 phase, which was prevented by treatment with montelukast. Notably, montelukast reduced expression of the senescence markers p53, p21 and PAI-1. In addition, montelukast ameliorated TNF-α-induced K382 acetylation of p53 by promoting the expression of SIRT1. Silencing of SIRT1 using SIRT1 siRNA broke the inhibitory effects of montelukast on K382 acetylation of p53. Importantly, silencing of cysLTR1 reversed the reduction of SIRT1 expression as well as the K382 acetylation of p53. Our findings strongly implicate that cysLTR1 has the capacity to regulate cellular senescence in chondrocytes. It is suggested that montelukast may be a potential therapeutic agent for chondro-protective therapy.
MITA H ET AL: "Levels of cysteinyl leukotriene receptor mRNA in humanperipheral leucocytes: significantly higher expression of cysteinyl leukotriene receptor 2 mRNA in eosinophils": Cysteinyl leukotrienes (CysLTs) have been implicated as important contributors in the pathophysiology of asthma and their biological effects are mediated by at least two distinct G-protein-coupled receptors. cDNA sequences of cysteinyl leukotriene receptor 1 (CysLTR1) and cysteinyl leukotriene receptor 2 (CysLTR2) have recently been elucidated. Our aim is to explore gene expression and the comparative expression of CysLTR1 mRNA and CysLTR2 mRNA in human peripheral blood leucocytes. Methods Gene expression of CysLTR1 and CysLTR2 mRNAs in human peripheral blood eosinophils, neutrophils, monocytes and T lymphocytes has been measured by competitive reverse transcription-polymerase chain reactions using RNA or DNA competitors.
SHIRASAKI H ET AL: "Agonist- and T"H2 cytokine-induced up-regulation of cysteinyl leukotriene receptor messenger RNA in human monocytes": The cysteinyl leukotrienes (CysLTs) are lipid mediators that have been implicated in the pathogenesis of allergic diseases, and their actions are mediated via specific receptors named CysLT1 receptor (CysLT1R) and CysLT2 receptor (CysLT2R). Little information is known about the role of TH2 cytokines in the regulation of both CysLT1R and CysLT2R expression. To investigate the possible modulation of both CysLT1R and CysLT2R messenger RNA (mRNA) expression, we have developed a real-time quantitative reverse transcription-polymerase chain reaction (RT-PCR) assay based on the TaqMan fluorescence method to quantify CysLT1R and CysLT2R mRNA in human monocytes.
WO 2006/024449 A2 provides a human CysLT1 which is associated with the cardiovascular diseases, dermatological diseases, endocrinoloigcal diseases, metabolic diseases, cancer, inflammation, gastroenterological diseases, hematological diseases, respiratory diseases, muscle skeleton diseases, neurological diseases, urological diseases. The invention also provides assays for the identification of compounds useful in the treatment or prevention of cardiovascular diseases, dermatological diseases, endocrinoloigcal diseases, metabolic diseases, cancer, inflammation, gastroenterological diseases, hematological diseases, respiratory diseases, muscle skeleton diseases, neurological diseases, urological diseases. The invention also features compounds which bind to and/or activate or inhibit the activity of CysLT1 as well as pharmaceutical compositions comprising such compounds.
NEGRIJ ET AL: "Corticosteroids as inhibitors of cysteinyl leukotriene metabolic and signaling pathways": Corticosteroids (CCSs) do not influence secretion of cysteinyl leukotrienes (CysLTs) that occurs on cellular activation during allergic reactions nor do they modulate bronchospastic responses to inhalation challenges with leukotrienes (LTs). We speculated that CCSs might modulate pathways responsible for CysLT production and diminish the ability of cellular activation to cause their release. Similarly, CCSs could reduce expression of CysLT receptor 1 (CysLTR1) and CysLT receptor 2 (CysLT2R) and modulate their responsiveness.
STEINKE JOHN W ET AL: "Characterization of interleukin-4-stimulated nasal polyp fibroblasts": Chronic hyperplastic eosinophilic sinusitis is an inflammatory disease that results in the accumulation of eosinophils, fibroblasts, mast cells, and goblet cells at the site of injury. A common feature of this disease is the presence of nasal polyposis (NP). The current studies were designed to assess the contribution of interleukin (IL)-4 to fibroblast-mediated inflammation in chronic hyperplastic eosinophilic sinusitis/NP. In addition, we hypothesized that cysteinyl leukotrienes (CysLT) may directly influence fibroblast-mediated fibrotic and remodeling pathways in this disorder. Fibroblasts were isolated from NP tissue. All fibroblast lines expressed the IL-4 receptor. IL-4 induced changes in mRNA and protein expression of fibrotic (transforming growth factor-β1 and -β2) and inflammatory cytokines and chemokines (IL-6 and CCL11) by fibroblasts as measured by semiquantitative and quantitative polymerase chain reaction, RNase protection assay, and enzyme-linked immunosorbent assay. The expression of CysLT and other proinflammatory lipid receptors on fibroblasts was evaluated. CysLT1 and CysLT2 receptors were not expressed on fibroblasts; however, LPA₁ receptor was constitutively expressed and LPA₂ receptor expression was upregulated by IL-4. The metabolic cascade involved in CysLT synthesis was not expressed in fibroblasts and could not be induced by IL-4 treatment.

### SUMMARY

The present application is defined by the appended claims.

The present disclosure provides a primer probe set for RT-PCR detection of a human leukotriene receptor *CYSLTRI* mRNA. In the present disclosure, a one-step detection is conducted based on the primer probe set without separate reverse transcription, which greatly reduces the risk of causing aerosol pollution. Compared with immunological detection methods, the detection method using the primer probe set of the present disclosure has high sensitivity, can detect low-concentration (10 copies/µL) clinical samples, can sensitively detect changes in CysLTR1 content, and has a detection range spanning at least 5 orders of magnitude. Accordingly, the accuracy of the detection results is increased, such that the treatment effect can be dynamically monitored and evaluated in an earlier, more accurate, and faster manner.

### BRIEF DESCRIPTION OF THE DRAWINGS

FIG. 1 is a process diagram of a dilution operation provided by the present disclosure.
FIG. 2 is a standard curve of a TaqMan real-time fluorescence quantitative RT-PCR for *CYSLTRI* mRNA provided by the present disclosure.
FIG. 3 is a result of a precision detection provided by the present disclosure. Herein, 1: 1.0×10⁷ copies/µL, and 2: 1.0×10⁴ copies/µL.
FIG. 4 is a result of an accuracy detection provided by the present disclosure.
FIG. 5 is a result of a sensitivity detection provided by the present disclosure.
FIG. 6 is a result of a clinical sample detection provided by the present disclosure. Herein, 1: patient *GAPDH* mRNA; 2: healthy control *GAPDH* mRNA; 3: patient *CYSLTR1* mRNA; and 4: healthy control *CYSLTRI* mRNA.
FIG 7 is a low-precision amplification curve in the case of an unreasonable primer design provided by the present disclosure.
FIG. 8 is an amplification result of an enzyme mixed solution (A) with the non-optimal ratio and an enzyme mixed solution (B) with the optimal ratio provided by the present disclosure.

### DETAILED DESCRIPTION OF THE EMBODIMENTS

The present disclosure provides a primer probe set for RT-PCR detection of a human leukotriene receptor *CYSLTRI* mRNA, including a primer CysLTR1-F, a primer CysLTR1-R and a probe C1-Probe, where the primer CysLTR1-F has a nucleotide sequence shown in SEQ ID NO. 1: 5'- AACCTATCACAAGAAGTCAGC-3', the primer CysLTR1-R has a nucleotide sequence shown in SEQ ID NO.2: 5'- CCAAAGAGCCAAATGCCTTT-3', and the probe C1-Probe has a nucleotide sequence shown in SEQ ID NO.3: 5'-CACTGCCTCTCCGTGTGGTC-3'.

In the present disclosure, a 5'-end of the probe C1-Probe is labeled with a fluorescent reporter group, and a 3'-end of the probe C1-Probe is labeled with a quenching group. The fluorescent reporter group preferably includes an FAM or a JOE, and the quenching group preferably includes a BHQ1. A 5'-end of the probe C1-Probe is labeled with an FAM fluorescent reporter group, and a 3'-end of the probe C1-Probe is labeled with a BHQ1 quenching group.

In the present disclosure, the primer probe set further includes a primer GAPDH-F, a primer GAPDH-R and a probe G-Probe of a reference gene, where the primer GAPDH-F may have a nucleotide sequence shown in SEQ ID NO.4: 5'-GACAACAGCCTCAAGATCATC-3', the primer GAPDH-R may have a nucleotide sequence shown in SEQ ID NO.5: 5'-CGCCACAGTTTCCCGGAG-3', and the probe G-Probe may have a nucleotide sequence shown in SEQ ID NO.6: 5'-ACTCATGACCACAGTCCATGCCAT-3'. A 5'-end of the probe G-Probe is labeled with a fluorescent reporter group, and a 3'-end of the probe G-Probe is labeled with a quenching group; and the fluorescent reporter group labeled on the probe G-Probe is preferably different from the fluorescent reporter group labeled on the probe C1-Probe. The fluorescent reporter group preferably includes the FAM or the JOE, and the quenching group preferably includes the BHQ1. In an example, a 5'-end of the probe G-Probe is labeled with a JOE fluorescent reporter group, and a 3'-end of the probe G-Probe is labeled with a BHQ1 quenching group.

The present disclosure further provides a kit for RT-PCR detection of a human leukotriene receptor *CYSLTRI* mRNA, including the primer probe set, a PCR reaction solution, an enzyme mixed solution, a CysLTR1 standard, an ROX reference dye and nuclease-free water.

In the present disclosure, the PCR reaction solution includes a deoxy-ribonucleoside triphosphate (dNTP) mix, MgCl₂ and a buffer; the dNTP mix includes a dATP, a dCTP, a dGTP and a dTTP; and the dNTP mix is preferably purchased from Thermo Fisher Scientific (product number: R0192), and has a working concentration of preferably 0.3~0.8 mM. The MgCl₂ has a concentration of preferably 5-12 mM during using; and the buffer is preferably a Tris-HCl buffer, more preferably a 20-50 mM Tris-HCl buffer with a pH of preferably 8.0.

In the present disclosure, the enzyme mixed solution includes a Taq enzyme, a reverse transcriptase, an RNase inhibitor and a Taq enzyme antibody with a volume ratio of preferably 15:5:3:1 to obtain the optimal amplification effect. The Taq enzyme is a heat-resistant Taq DNA polymerase, deoxynucleotides in the dNTP are added to a 3-OH terminus one by one using the 3'→5' polymerase activity of the Taq enzyme and using DNA as a template. Meanwhile, mismatched primer ends can be identified and eliminated using the 5'→3' exonuclease activity of the Taq enzyme, which is related to the correction function during the replication, nucleotides can also be hydrolyzed from the 5'-end and mismatched nucleotides can also be excised through several nucleotides. In this way, the chain replacement is realized during the chain extension, and the replaced probe is cut off. The reverse transcriptase can reverse transcribe an mRNA into a cDNA for PCR reaction. The RNase inhibitor is used to suppress the activity of an exogenous RNase. The Taq enzyme antibody is an anti-Taq antibody for hot-start PCR, inhibits DNA polymerase activity after binding to the Taq enzyme, and can effectively suppress the non-specific annealing of primers and the non-specific amplification caused by primer dimers under low temperature. The Taq enzyme antibody is denatured during the initial DNA denaturation of the PCR reaction, and the Taq enzyme recovers the activity to realize PCR amplification. The CysLTR1 standard is preferably a RNA standard of the CysLTR1 for preparing a quantitative curve.

The present disclosure further provides a method for using the kit, including the following steps: mixing the primer probe set, the PCR reaction solution, the enzyme mixed solution, the CysLTR1 standard or a CysLTR1 sample to be tested, the ROX reference dye and the nuclease-free water, and conducting fluorescence quantitative amplification. In the present disclosure, the kit adopts a quantitative detection method of one-step RT-PCR technology, which can detect an expression level of the *CYSLTRI* mRNA in human blood.

In the present disclosure, based on 20 µL, a reaction system of the kit includes: 2 µL of the primer probe set, 10 µL of the PCR reaction solution, 0.5 µL of the enzyme mixed solution, 0.1 µL of the ROX reference dye, 5 µL of the CysLTR1 standard or the CysLTR1 sample to be tested, and 2.4 µL of the nuclease-free water. The fluorescence quantitative amplification is preferably conducted by: 42°C for 30 min (reverse transcription); 95°C for 1 min (pre-denaturation); 95°C for 5 s, and 60°C for 31 s, for 40 cycles.

In the present disclosure, the kit has a simple operation and short detection time. The present disclosure provides a kit product that can guide the medication and accurately quantify the efficacy for CysLTR1 receptor antagonists. Cysteinyl leukotrienes (CysLTs) are inflammatory mediators and regulators in the pathophysiology of asthma and allergic rhinitis (AR), and are key therapeutic targets. During treatment, leukotriene receptor CysLTR1 antagonists can reduce allergic inflammations by blocking the activity of CysLTR1, and produce broad clinical effects. The expression of the leukotriene receptor *CYSLTRI* mRNA is detected to be higher than the normal reference range, indicating that the treatment on patients will be effective with a leukotriene receptor CysLTR1 antagonist; and the level of CysLTR1 in the blood is detected to be lowered after treatment, indicating that the treatment is effective. If obvious allergic symptoms are shown, but the expression of leukotriene receptor CysLTR1 is very low; this indicates that the allergic symptoms are not caused by leukotriene pathway, and the treatment with leukotriene receptor CysLTR1 antagonist is invalid.

The primer probe set and the kit for RT-PCR detection of a human leukotriene receptor *CYSLTRI* mRNA according to the present disclosure will be further described in detail below with reference to specific examples. The technical solutions of the present disclosure include, but are not limited to, the following examples.

Unless otherwise specified, the experimental methods described in the following examples are all conventional methods. The methods shall be conducted in accordance with the techniques or conditions described in the literature in the art or in accordance with the product specification. The materials and reagents and the like used in the following examples are all commercially available, unless otherwise specified.

### Example 1

1. The reagents and equipment involved were as follows:
   1.1 Reagents
   1.1.1 A whole-blood total RNA kit (Hangzhou Simgen Biological Reagent Development Co., Ltd., product number: 5201050).
   1.1.2 A HiScribe T7 High Yield RNA Synthesis Kit (New England Biolabs, product number: E2040S).
   1.2 Main instruments
   1.2.1 An Applied Biosystems^{™} 7300 fluorescence quantitative PCR instrument (Thermo Fisher Scientific, USA).
   1.2.2 A -80°C low-temperature refrigerator (Thermo Fisher Scientific, USA).
   1.2.3 A high-speed and low-temperature table centrifuge (Eppendorf, Germany).
   1.2.4 A Qubit 3 fluorometer (Thermo Fisher Scientific, USA).

### 2. Method

### 2.1 Design of primers and probes

Fluorescent quantitative primers and probes were designed using a Primer 6.0 software according to the sequence of a CysLTR1 and a GAPDH; after a series of effect verification, primer pairs CysLTR1-F, CysLTR1-R, GAPDH-F, and GAPDH-R and probes E-Probe, and G-Probe of the CysLTR1 and the GAPDH were obtained (Table 1). The primers and probes were synthesized by Shanghai Sunny Biotechnology Co., Ltd.

**Table 1 TaqMan real-time fluorescence quantitative PCR of primers and probes**

| Name of primers and probes | Use | Primer sequence (5'→3') (SEQ ID NO.) | Amplified fragment size |
|---|---|---|---|
| CysLTR1-F | Fluorescence quantitative amplification of *CYSLTR1* cDNA fragment | AACCTATCACAAGAAGTCAGC (1) | 126 bp |
| CysLTR1-R | | CCAAAGAGCCAAATGCCTTT (2) | |
| C1-Probe | | (FAM)-CACTGCCTCTCCGTGTGGTC (3)-(BHQ1) | |
| GAPDH-F | Fluorescence quantitative amplification of reference gene *GAPDH* fragment | GACAACAGCCTCAAGATCATC (4) | 70 bp |
| GAPDH-R | | CGCCACAGTTTCCCGGAG (5) | |
| G-Probe | | (JOE)-ACTCATGACCACAGTCCATGCCAT (6)-(BHQ1) | |

### 2.2. Preparation of a standard

In-vitro transcription: a pGM-T ligation kit [TIANGEN Biotech (Beijing) Co., Ltd., product number: VT202-01] was used, a CysLTR1 plasmid DNA (constructed and synthesized by entrusting Nanjing GenScript Biotech Co., Ltd.) was constructed using a pGM-T as a vector, and the CysLTR1 plasmid DNA was transcribed into an mRNA in vitro using a HiScribe T7 High Yield RNA Synthesis Kit (NEW ENGLAND BioLabs, product number: E2040S).

An initial copy number of RNA was calculated according to a copy number calculation formula: copy number=[6.02×10²³×RNA concentration (ng/µL)×10⁻⁹]/[RNA length (bp)×340]. The *CYSLTRI* mRNA was diluted with nuclease-free water to 1.0×10¹⁰ copies/µL to obtain a *CYSLTRI* mRNA standard.

2.3. Extraction and dilution of a whole-blood RNA: the whole-blood total RNA was extracted from ethylenediaminetetraacetic acid (EDTA) anticoagulated whole-blood samples with the whole-blood total RNA kit, quantificated with the Qubit 3 fluorometer and diluted with the nuclease-free water to 20 ng/µL.

### 2.4 TaqMan real-time fluorescent quantitative PCR

A 20 µL of system was prepared using the *CYSLTRI* mRNA standard or the whole blood RNA as a template, the system was shown in Table 2:

**Table 2 Reaction system**

| Nuclease-free water | PCR reaction solution | Enzyme mixed solution | ROX reference dye | Primer-probe mixed solution | *CYSLTRI* mRNA Standard or whole-blood RNA | Total volume |
|---|---|---|---|---|---|---|
| 2.4 µL | 10 µL | 0.5 µL | 0.1 µL | 2 µL | 5 µL | 20 µL |

An amplification reaction program was shown in Table 3:

**Table 3 Reaction program**

| Stage | Temperature | Time | Number of cycles | Other parameter settings |
|---|---|---|---|---|
| Stage 1 | 42°C | 30 min | 1 | Detection fluorescein: FAM, JOE |
| Stage 2 | 95°C | 1 min | 1 | Reference fluorescence: ROX |
| Stage 3 | 95°C | 5 sec | 40 | Reaction system: 20 µL |
| | 60°C | 31 sec | | Fluorescence signal collection: Stage 3, 60°C for 31 sec |

### 2.5 Generation of a standard curve

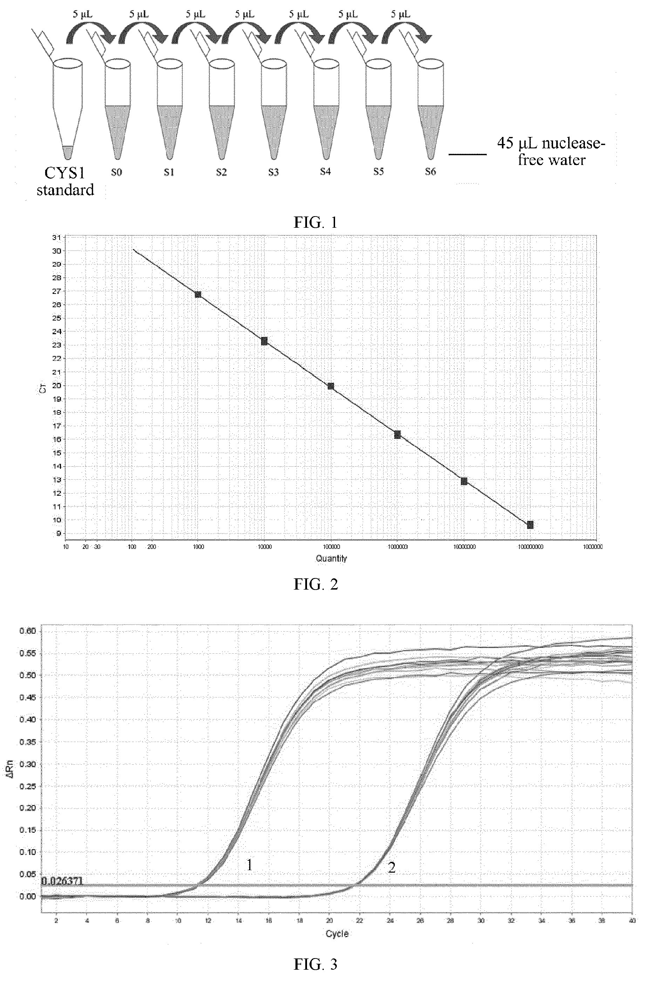

The *CYSLTRI* mRNA standard was diluted in a 10-fold gradient using 1.0×10⁸-1.0×10³ copies/µL as a template, 3 replicates were conducted for each dilution, and TaqMan real-time fluorescence quantitative RT-PCR detection was conducted to generate a standard curve. The dilution operation was shown in FIG. 1. A 50 µL/tube was taken as an example: for each dilution, 5 µL of a sample before dilution was added to a new tube containing 45 µL of water.

### 2.6 Precision detection

1.0×10⁷ copies/µL and 1.0×10⁴ copies/µL of *CYSLTRI* mRNA standards were taken as a template, 10 replicates were conducted for each concentration; 10 times of TaqMan real-time fluorescent quantitative RT-PCR detections were conducted, the coefficient of variation of the logarithm of each concentration was calculated, respectively; and statistical analysis was conducted to analyze the precision of the detection method.

### 2.7 Accuracy detection

A 1.0×10⁶ copies/µL of *CYSLTRI* mRNA standard was subjected to 30-fold dilution (2 µL 1.0×10⁶ copies/µL of a *CYSLTRI* mRNA standard + 58 µL of nuclease-free water) as a template, for 3 replicates; 3 times of TaqMan real-time fluorescence quantitative RT-PCR detections were conducted, and the accuracy of the detection method was analyzed.

### 2.8 Sensitivity detection

A 10.0 copies/µL of *CYSLTRI* mRNA standard was taken as a template, for 25 replicates, 25 times of TaqMan real-time fluorescence quantitative RT-PCR detection were conducted to check whether there were amplifications, and the sensitivity of the detection method was analyzed.

### 2.9 Clinical sample detection

Whole-blood samples of positive samples and healthy control were taken to extract and dilute whole-blood RNA according to the steps of 2.3; TaqMan real-time fluorescent quantitative RT-PCR detection was conducted according to the steps of 2.4.

### 3. Experimental results

### 3.1 Standard curve

The *CYSLTRI* mRNA standard was diluted in a 10-fold gradient using 1.0×10⁸-1.0×10³ copies/µL as a template, 3 replicates were conducted for each dilution, and TaqMan real-time fluorescence quantitative RT-PCR detection was conducted to generate a standard curve. The standard curve of the TaqMan real-time fluorescence quantitative RT-PCR of the *CYSLTR1* mRNA is shown in FIG. 2. A copy number logarithm was taken as an abscissa and a Ct value is taken as an ordinate, and a regression equation was obtained: y=-3.398x+35.344 (R²=1.000), where the regression equation has R²=1.000, and a linear range of 1.0×10³-1.0×10⁸ copies/µL. It indicates that the copy number logarithm of the standard equation has a very high correlation with the Ct value.

### 3.2 Precision detection

1.0×10⁷ copies/µL and 1.0×10⁴ copies/µL of *CYSLTRI* mRNA standards were taken as templates, 10 replicates were conducted for each concentration; 10 times of TaqMan real-time fluorescent quantitative RT-PCR detections were conducted, the coefficient of variation of the logarithm of each concentration was calculated, respectively; and statistical analysis was conducted. The results are shown in FIG. 3 and Table 4. The coefficient of variation of the logarithm of each concentration is 0.320% and 0.444% separately, which are less than 5%, indicating that the TaqMan real-time fluorescent quantitative RT-PCR detection method established by the present disclosure has excellent precision.

**Table 4 Precision detection result**

| Theoretical copy number | Mean of copy number logarithm | SD | C.V |
|---|---|---|---|
| 1.0×10⁷ | 7.042 | 0.023 | 0.320% |
| 1.0×10⁴ | 3.996 | 0.018 | 0.444% |

### 3.3 Accuracy detection

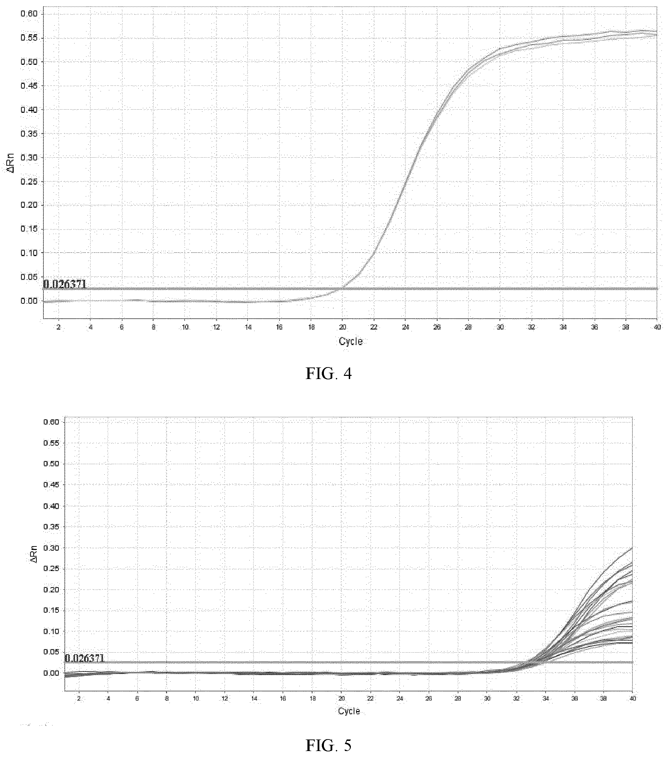

A 1.0×10⁶ copies/µL *CYSLTRI* mRNA standard was subjected to 30-time dilution (2 µL 1.0×10⁶ copies/µL of a *CYSLTRI* mRNA standard + 58 µL of nuclease-free water) as a template, for 3 replicates; 3 times of TaqMan real-time fluorescence quantitative RT-PCR detections were conducted, and the absolute deviation of the logarithm of each concentration was calculated. The results are shown in FIG. 4 and Table 5. The absolute deviation of the logarithm of each concentration is 0.002, 0.011, and 0.008, respectively, within the range of ±0.5, indicating that the TaqMan real-time fluorescent quantitative RT-PCR detection method established by the present disclosure has excellent accuracy.

**Table 5 Accuracy detection result**

| C_{T} | Results (copies/µL) | Copy number logarithm | Theoretical copy number (copies/µL) | Theoretical copy number logarithm | Absolute deviation |
|---|---|---|---|---|---|
| 19.967 | 3.347×10⁴ | 4.525 | 3.333×10⁴ | 4.523 | 0.002 |
| 19.935 | 3.422×10⁴ | 4.534 | | | 0.011 |
| 19.946 | 3.395×10⁴ | 4.531 | | | 0.008 |

### 3.4 Sensitivity detection

A 10.0 copies/µL of *CYSLTRI* mRNA standard was taken as a template, for 25 replicates, 25 times of TaqMan real-time fluorescence quantitative RT-PCR detection were conducted to check whether there were amplifications. The results are shown in FIG. 5 and Table 6. A total of 25 detection results are obtained, reaching 100%. This indicates that the TaqMan real-time fluorescent quantitative RT-PCR detection method established by the present disclosure has very high sensitivity, and the minimum of detected copy number is less than 10 copies/µL.

**Table 6 Ct value result of sensitivity detection**

| | | | | |
|---|---|---|---|---|
| 33.271 | 33.123 | 33.228 | 32.975 | 33.800 |
| 33.150 | 33.284 | 32.547 | 33.408 | 33.416 |
| 33.270 | 33.216 | 34.086 | 32.939 | 32.709 |
| 33.312 | 33.788 | 33.070 | 33.653 | 32.923 |
| 32.789 | 33.700 | 33.624 | 33.562 | 32.810 |

### 3.5 Clinical sample detection

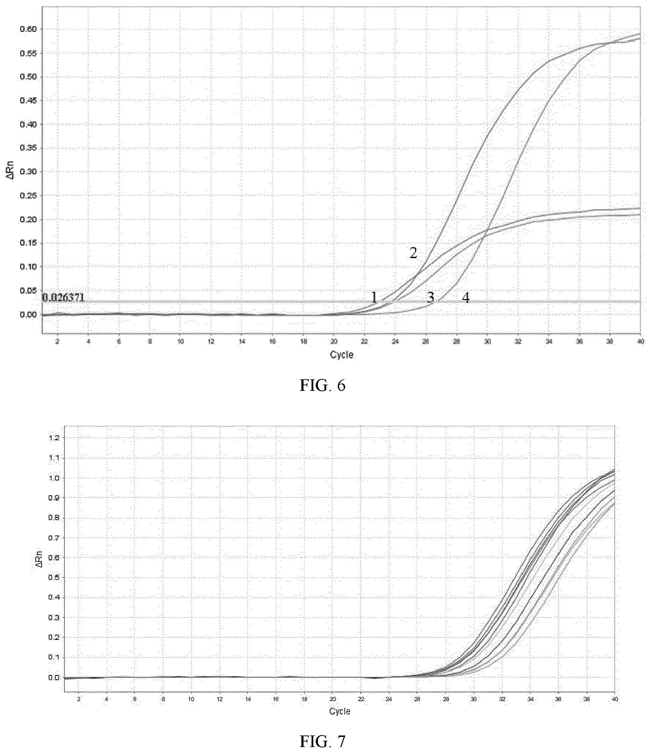

A comparison result of the present disclosure and a certain domestic brand of cysteinyl leukotriene receptor 1 (CYSLTR1) kit of enzyme-linked immunosorbent assay (ELISA) are shown in FIG. 6 and Table 7.

**Table 7 Comparison result**

| SN | Sample type | Product of the present disclosure | | CYSLTR1 kit of ELISA | |
|---|---|---|---|---|---|
| | | Results (copies/µL) | positive/negative | Results (ng/mL) | positive/negative |
| **1** | Sample 1 before treatment | 1589.924 | + | 232.6 | + |
| **2** | Sample 1 after treatment | 87.802 | - | 52.4 | - |
| **3** | Sample 2 before treatment | 2432.674 | + | 353.8 | + |
| **4** | Sample 2 after treatment | 341.476 | - | 155.7 | + |
| **5** | Sample 3 before treatment | 1543.690 | + | 274.2 | + |
| **6** | Sample 3 after treatment | 96.472 | - | 80.7 | - |
| **7** | Positive sample 4 | 3434.793 | + | 612.3 | + |
| **8** | Healthy control 4 | 38.530 | - | 72.4 | - |
| **9** | Healthy control 5 | 123.466 | - | 176.7 | + |
| **10** | Healthy control 6 | 67.019 | - | 49.9 | - |

In the present disclosure, the detection was conducted using the whole-blood RNA; and the certain domestic brand of CYSLTR1 kit of ELISA was detected using a serum.

### Comparative Example 1

### Results of amplification using other non-optimal primers and probes

The primers and probes in the system used in the present disclosure were replaced with other non-optimal primers and probes. An amplification system and a program were the same as those in Example 1. The results are shown in FIG. 7 and Table 8. When non-optimal CysLTR1 primers and probes are used, such as:
CysLTR1-F: GTATCTTCTGCCACATGCC (SEQ ID NO. 7);
CysLTR1-R: TTGCCAAAGAAGCCTACAACA (SEQ ID NO. 8); and
C1-Probe: (FAM)-CCGCAATCAAGTGTATTCCACC (SEQ ID NO. 9)-(BHQ1).

The coefficient of variation of the logarithm of the low-precision concentration exceeds 5%, reaching 8.813%.

**Table 8 Results of amplification with non-optimal primers and probes**

| Theoretical copy number | Mean of copy number logarithm | SD | C.V |
|---|---|---|---|
| 1.0×10⁴ | 3.637 | 0.321 | 8.813% |

### Comparative Example 2

### Amplification result of non-optimal enzyme mixed solution

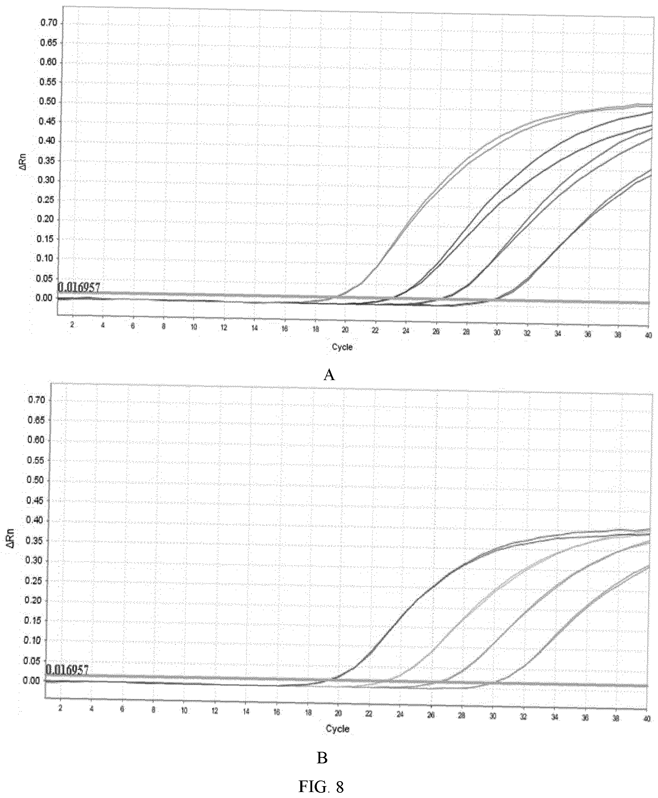

An amplification was conducted using a non-optimal ratio of enzyme mixed solution (the Taq enzyme, reverse transcriptase, RNase inhibitor and Taq enzyme antibody had a mass ratio of 14:4:5: 1) and an optimal ratio of enzyme mixed solution on the *CYSLTR1* mRNA standard with the primers and probes, the amplification system, and the program the same as those in Example 1. 4 gradients 1.0×10³-1.0×10⁶ copies/µL of a standard curve were obtained, and the result is shown in FIG. 8. An amplification result using the non-optimal ratio of enzyme mixed solution is shown in FIG. 8A, and an amplification result using the optimal ratio of enzyme mixed solution is shown in FIG. 8B. In a comparison, a standard curve of the amplification result using the optimal enzyme mixed solution has better repeatability, and has Ct differences between adjacent concentrations of 3.3, 3.3, and 3.4, respectively; meanwhile, a standard curve of the amplification result using the non-optimal enzyme mixed solution has Ct differences between adjacent concentrations of 3.8, 3.1, and 3.6, respectively. It indicates that the Ct difference of the standard curve of the amplification result using the optimal enzyme mixed solution is more uniform. It can be seen that the optimal enzyme mixed solution has better amplification effect.

The above results show that the TaqMan real-time fluorescent quantitative RT-PCR detection method established in the present disclosure has better sensitivity and specificity than that of a counterpart reagent, and can effectively monitor the treatment effect.

The above descriptions are merely preferred implementations of the present disclosure. It should be noted that a person of ordinary skill in the art may further make several improvements and modifications without departing from the principle of the present disclosure, but such improvements and modifications should be deemed as falling within the protection scope of the present disclosure.

## Claims

1. A kit for one-step RT-PCR detection of a human leukotriene receptor *CYSLTRI* mRNA, comprising a primer probe set comprising a probe C1-Probe that has a nucleotide sequence shown in SEQ ID NO.3, a PCR reaction solution, an enzyme mixed solution, a CysLTR1 standard, a carboxy-X-rhodamine (ROX) reference dye and nuclease-free water;
**characterized in that**
the primer probe set further comprises a primer CysLTR1-F and a primer CysLTR1-R, wherein the primer CysLTR1-F has a nucleotide sequence shown in SEQ ID NO. 1, and the primer CysLTR1-R has a nucleotide sequence shown in SEQ ID NO.2;
the enzyme mixed solution comprises a thermus aquaticus (Taq) enzyme, a reverse transcriptase, a ribonuclease (RNase) inhibitor and a Taq enzyme antibody at a volume ratio of 15:5:3:1.

2. The kit according to claim 1, wherein the PCR reaction solution comprises a deoxy-ribonucleoside triphosphate (dNTP) mix, MgCl₂ and a buffer.

3. A method for using the kit according to any one of claims 1-2, comprising the following steps: mixing the primer probe set, the PCR reaction solution, the enzyme mixed solution, the standard or a sample to be tested, the ROX reference dye and the nuclease-free water, and conducting fluorescence quantitative amplification.

4. The method according to claim 3, wherein based on 20 µL, a reaction system of the kit comprises: 2 µL of the primer probe set, 10 µL of the PCR reaction solution, 0.5 µL of the enzyme mixed solution, 0.1 µL of the ROX reference dye, 5 µL of the standard or the sample to be tested, and 2.4 µL of the nuclease-free water; and
the fluorescence quantitative amplification is conducted by: 42°C for 30 min; 95°C for 1 min; 95°C for 5 s, and 60°C for 31 s, 40 cycles.

## Patentansprüche

1. Ein Kit für den einstufigen RT-PCR-Nachweis einer humanen Leukotrienrezeptor-*CYSLTR1*-mRNA, bestehend aus einem Primer-Sonden-Set mit einer C1-Sonde, deren Nukleotidsequenz in SEQ ID NR.3 dargestellt ist, einer PCR-Reaktionslösung, einer Enzymmischungslösung, einem CysLTR1-Standard, einem Carboxy-X-Rhodamin (ROX)-Referenzfarbstoff und nukleasefreiem Wasser;
**dadurch gekennzeichnet, dass**
das Primer-Sonden-Set ferner einen Primer CysLTR1-F und einen Primer CysLTR1-R umfasst, wobei der Primer CysLTR1-F eine Nukleotidsequenz aufweist, die in SEQ ID NR. 1 dargestellt ist, und der Primer CysLTR1-R eine Nukleotidsequenz aufweist, die in SEQ ID NR. 2 dargestellt ist;
die Enzymmischungslösung aus einem Thermus aquaticus (Taq)-Enzym, einer reversen Transkriptase, einem Ribonuklease (RNase)-Inhibitor und einem Taq-Enzym-Antikörper im Volumenverhältnis 15:5:3:1 besteht.

2. Das Kit nach Anspruch 1, wobei die PCR-Reaktionslösung eine Desoxyribonukleosidtriphosphat-Mischung (dNTP), MgCl₂ und einen Puffer enthält.

3. Ein Verfahren zur Verwendung des Kits nach einem der Ansprüche 1 oder 2, umfassend die folgenden Schritte: Mischen des Primer-Sonden-Sets, der PCR-Reaktionslösung, der Enzymmischungslösung, des Standards oder einer zu testenden Probe, des ROX-Referenzfarbstoffs und des nukleasefreien Wassers und Durchführung einer quantitativen Fluoreszenzamplifikation.

4. Das Verfahren nach Anspruch 3, wobei ein Reaktionssystem des Kits, basierend auf 20 µL, Folgendes umfasst: 2 µL des Primer-Sonden-Sets, 10 µL der PCR-Reaktionslösung, 0,5 µL der Enzymmischung, 0,1 µL des ROX-Referenzfarbstoffs, 5 µL des Standards oder der zu testenden Probe und 2,4 µL des nukleasefreien Wassers; und
die quantitative Fluoreszenzverstärkung wie folgt durchgeführt wird: 42 °C für 30 Min.; 95 °C für 1 Min.; 95 °C für 5 s und 60 °C für 31 s, 40 Zyklen.

## Revendications

1. Un kit pour la détection RT-PCR en une étape d'un récepteur des leucotriènes humains *CYSLTR1* ARNm, comprenant un ensemble de sondes amorces comprenant une sonde C1 avec une séquence de nucléotides présentée dans SEQ ID N° 3, une solution de réaction PCR, une solution enzymatique mixte, un étalon CysLTR1, un colorant de référence carboxy-X-rhodamine (ROX) et de l'eau sans nucléase ;
**caractérisé en ce que**
l'ensemble de sondes amorces comprend en outre une amorce CysLTR1-F et une amorce CysLTR1-R, l'amorce CysLTR1-F possédant une séquence de nucléotides indiquée dans SEQ ID N° 1, et l'amorce CysLTR1-R possédant une séquence de nucléotides indiquée dans SEQ ID N° 2 ;
la solution enzymatique mixte comprend une enzyme thermus aquaticus (Taq), une transcriptase inverse, un inhibiteur de ribonucléase (RNase) et un anticorps enzymatique Taq avec un rapport volumique de 15:5:3:1.

2. Kit selon la revendication 1, dans lequel la solution de réaction PCR comprend un mélange de triphosphate désoxyribonucléoside (dNTP), MgCl₂ et un tampon.

3. Procédé d'utilisation du kit selon l'une des revendications 1 à 2, comprenant les étapes suivantes : mélanger le jeu de sondes amorce, la solution de réaction PCR, la solution enzymatique mixte, l'étalon ou un échantillon à tester, le colorant de référence ROX et l'eau sans nucléase, et conduire l'amplification quantitative par fluorescence.

4. Procédé selon la revendication 3, dans lequel sur la base de 20 µL, un système de réaction du kit comprend : 2 µL de l'ensemble de sondes amorces, 10 µL de la solution de réaction PCR, 0,5 µL de la solution enzymatique mixte, 0,1 µL du colorant de référence ROX, 5 µL de l'étalon ou de l'échantillon à tester, et 2,4 µL de l'eau sans nucléase ; et
l'amplification quantitative par fluorescence est réalisée par à 42° C pendant 30 minutes ; 95° C pendant 1 minute ; 95° C pendant 5 s, et 60° C pendant 31 s, 40 cycles.
